# EUROPEAN PATENT APPLICATION

(11) **EP 4 744 684 A1**
(43) Date of publication of application: **20.05.2026**
(21) Application number: 25215165.9
(22) Date of filing: 12.11.2025
(51) Int. Cl.: A61L 2/18, B08B 9/08, C11D 3/39, E04H 4/16

(54) **KIT FOR CLEANING SWIMMING POOL WALLS MADE OF PLASTIC OR COMPOSITE PLASTIC MATERIAL**

(30) Priority: 14.11.2024 IT 202400025695
(71) Applicant: LAPI CHIMICI SOCIETA' PER AZIONI, 52048 Monte San Savino (AR) (IT)
(72) Inventor: DE LAPI, Lucia, 52048 MONTE SAN SAVINO (AR) (IT)
(74) Representative: Studio Torta S.p.A.

(57) **Abstract**

A kit for cleaning the yellow/brown line on the plastic material walls of a swimming pool at the waterline.

The kit comprises:
- a first container containing a liquid comprising (i) an acidic compound having a pKa at 25°C less than or equal to 5 at a concentration greater than or equal to 15% by weight and/or (ii) a liquid organic compound belonging to the class of ketones or glycols or acetates;
- a second container containing an aqueous solution comprising sodium hypochlorite at a concentration greater than or equal to 3% w/V.

## Description

### Cross-reference to related applications

This patent application claims priority from Italian patent application no. 102024000025695 filed on November 14, 2024, the entire disclosure of which is incorporated herein by reference.

### Technical field of the invention

The invention relates to a kit for the treatment of the plastic material walls of swimming pools for the elimination or significant reduction of the yellow/brown line that is formed at the waterline. In particular, the kit according to the invention comprises the combination of two solutions, whose sequential use guarantees the cleaning of the aforementioned yellow/brown line.

### State of the art

As it is known, for many years the walls of swimming pools have sometimes been lined with a layer of plastic material. To date, the plastic materials used to line swimming pools have a quality that also meets the required style requirements. To this regard, the most common plastic material used to line swimming pools is PVC, which is applied in the form of a liner with a thickness of a few mm and is very flexible. In order to improve the characteristics thereof in terms of resistance to UV rays and moulds, the PVC layer is subjected to particular treatments.

Although the plastic walls are ideal in many ways, they however present, more than other materials, a problem related to the formation of the yellow/brown line that forms on the side walls at the waterline.

This yellow/brown line is due to hyperpigmentation and to deposits of various kinds that, over time, form on the surface of the plastic material at the waterline.

Such a phenomenon is more evident in swimming pools maintained with chlorine and filled with well water, which is richer in metals and mineral salts than in tap water.

In addition, in pools with many swimmers, the effect of sunscreens also becomes significant. In fact, the accumulation of oils and sunscreens in the water, due to their dissolution, further amplifies the pigmentation of the surface and therefore the formation of stains, i.e. the yellow/brown line.

Last but not least, it has been observed that the stains occur more frequently in those areas of the pool surface that are more often exposed to sunlight. As a matter of fact, it should be considered that heat causes the plastic material to expand, thereby facilitating the penetration of residues into it.

On the market there are several products that are generally used to eliminate the yellow/brown line mentioned above.

However, these products are not effective in removing this specific yellow/brown line, as they are either simple degreasers that only remove greasy deposits from oils and sunscreens or descaling agents that merely eliminate lime scale deposits.

Therefore, it is necessary to have a product capable of eliminating or at least significantly reducing the yellow/brown line considered above.

### Description of the invention

The inventors of this invention found a treatment method for cleaning the yellow/brown line, said method providing the application on the stain of a solution of an acidic and/or of a liquid organic compound comprised in the class of ketones or glycols or acetates and, subsequently, of a solution of sodium hypochlorite. This method is commercially embodied in a kit consisting of at least two containers containing the solution of an acidic and/or of a liquid organic compound comprised in the class of ketones or glycols or acetates and the solution of sodium hypochlorite, respectively.

Object of the present invention is a kit for cleaning the yellow/brown line on the plastic material walls of a swimming pool at the waterline; said kit being characterized in that it comprises:
- a first container containing a liquid comprising (i) an acidic compound having a pKa at 25°C less than or equal to 5 at a concentration greater than or equal to 15% by weight and/or (ii) a liquid organic compound belonging to the class of ketones or glycols or acetates;
- a second container containing an aqueous solution comprising sodium hypochlorite at a concentration greater than or equal to 3% w/V.

Preferably, sodium hypochlorite is present in the solution at a concentration from 3% to 50% w/V.

Preferably, said acidic compound is an inorganic acid.

Preferably, the acidic compound is comprised in the group consisting of sulfuric acid, fluoroboric acid, hydrochloric acid, citric acid, ascorbic acid and nitric acid.

Preferably, said liquid organic compound is ethyl acetate.

Preferably, the first container comprises an aqueous solution comprising from 15 to 60 % by weight of said acidic compound and from 1 to 50 % by weight of said organic compound; more preferably, from 25 to 55% by weight of said acidic compound and from 5 to 40 % by weight of said organic compound.

Preferably, said first container comprises viscosifiers to allow its application by spreading.

A further object of the present invention is a method for cleaning the yellow/brown line formed at the waterline on plastic material walls of a swimming pool; said method being characterized in that it comprises in sequence:
- a first application operation, in which (i) a solution of an acidic compound having a pKa at 25°C less than or equal to 5 at a concentration greater than or equal to 15% by weight and/or (ii) a liquid organic compound belonging to the class of ketones or glycols or acetates is applied on an application area including a yellow/brown line formed on plastic material walls of a swimming pool, and
- a second application operation, in which a solution comprising sodium hypochlorite at a concentration greater than or equal to 3% w/V is applied over said application area.

### Preferred Embodiments of the Invention

Embodiments of the present invention are set forth below for purposes of illustration and not disclosure.

### - Combination 1 -

A first (Solution A) and a second (Solution B) aqueous solution were made.

### - Solution A

850.0 g of a 50% by weight solution of fluoroboric acid (HBF4) were weighed into a beaker with a volume of 1000 ml. The solution was stirred by means of a magnetic anchor and a stirrer, on which the beaker was placed.

150 g of pure ethyl acetate (CH3COOCH2CH3) were weighed into a 250 ml beaker and were then gradually added to the solution of fluoroboric acid kept under stirring at about 100 rpm. Once the addition was complete, the solution formed was kept under stirring for at least one minute.

The Solution A thus made is characterized by 42.5% by weight of fluoroboric acid and 15% by weight of ethyl acetate.

### - Solution B

1000 g of a 15% w/v solution of sodium hypochlorite (NaClO) were weighed into a beaker with a volume of 1000 ml.

### - Treatment application

To verify the effectiveness of the combination of the two solutions described above, we proceeded with the sequential application of the two solutions on a sample of PVC liner used for lining pool walls and on which the yellow/brown line considered above had formed.

Solution A was evenly distributed on the surface to be treated of the PVC liner. The distribution of solution A took place by means of a sprayer.

Subsequently, solution B was applied on the same surface on which solution A was applied. The distribution of solution B took place by means of a sprayer.

In this way, it was verified that the yellow/brown line, after only a first application of the two solutions, is significantly reduced.

For a complete elimination of the yellow/brown line, it was sufficient to repeat the sequential application of the two solutions a second time.

Alternatively, the distribution of solution A can be carried out by spreading with a brush.

### - Combinations 2- 6 -

For experimental completeness, five further combinations (Combinations 2 - 6) of the two aqueous solutions of the kit according to the invention were prepared and tested.

For the sake of simplicity, Table I shows the features of the solutions prepared.

**Table I**

| | Solution A | Solution B |
|---|---|---|
| Combination 2 | 15% by weight of sulfuric acid | 15% w/V of sodium hypochlorite |
| Combination 3 | 15% by weight of ascorbic acid | 15% w/V of sodium hypochlorite |
| Combination 4 | 15% by weight of citric acid | 15% w/V of sodium hypochlorite |
| Combination 5 | 36% by weight of sulfuric acid | 4.5% w/V of sodium hypochlorite |
| Combination 6 | Ethyl acetate | 15% w/V of sodium hypochlorite |

Combinations 2 - 6 were applied to the surface of the PVC liner in which the yellow/brown line was present by repeating the same procedure described above in relation to combination 1.

Combinations 2 - 6 demonstrated efficacy in removing the yellow/brown line.

In particular, combination 6 showed that the combined application of ethyl acetate and sodium hypochlorite is also able to guarantee the cleaning of the yellow/brown line.

The results of the tests of combinations 1 - 6 described above showed that fluoroboric acid and the presence of ethyl acetate in solution A (Combination 1) guarantee the best results in terms of time and degree of elimination. In particular, it is the opinion of the inventors that ethyl acetate increases the effectiveness of the cleaning as it causes a softening of the plastic and, therefore, a greater penetration of the chemical agents into the material to be cleaned.

### - Comparison -

For comparison purposes, tests were also performed with solution A alone and solution B alone. That is, not in combination with one another.

In particular, three formulations were prepared:
Comparison 1 - only solution A consisting of 85% of 50% fluoroboric acid (concentration of fluoroboric acid in the final solution corresponding to 42.5%) and 15% of pure (100%) ethyl acetate;
Comparison 2 - only solution A consisting of 70% of 50% fluoroboric acid (concentration of fluoroboric acid in the final solution corresponding to 35%) and 30% of pure (100%) anhydrous ethyl acetate; and
Comparison 3 - only solution B consisting of 100% of 18% sodium hypochlorite.

Each of the comparison formulations set forth above was tested individually to verify its effectiveness in cleaning the yellow/brown line.

The application was carried out by spraying with a manual trigger. Sufficient time was then waited to observe any removal effects.

None of the three comparisons led to the removal (visible elimination) of the stain, which, in fact, remained visible even after the application of each solution (applied individually) .

Consequently, the use of the sole solution A (even in different formulations) or of the sole solution B is not able to effectively remove the yellow/brown stain, thus highlighting the synergistic activity of the two when applied together. In other words, only the combined and sequential use of solution A and solution B is capable of ensuring the removal of the yellow/brown stain.

As far as it was possible to verify experimentally, the kit of the invention guarantees a rapid and resolutive action for the removal of the yellow/brown line.

Finally, it is extremely important to point out that the kit of the invention allows the walls to be treated even in the presence of water in the pool. This, as one can easily understand, represents a significant advantage in terms of productivity for pool managers.

## Claims

1. A kit for cleaning the yellow/brown line on the plastic material walls of a swimming pool at the waterline; said kit being **characterized in that** it comprises:
- a first container containing a liquid comprising (i) an acidic compound having a pKa at 25°C less than or equal to 5 at a concentration greater than or equal to 15% by weight and/or (ii) a liquid organic compound belonging to the class of ketones or glycols or acetates;
- a second container containing an aqueous solution comprising sodium hypochlorite at a concentration greater than or equal to 3% w/V.

2. The kit according to claim 1, **characterized in that** sodium hypochlorite is present in the solution at a concentration from 3% to 50% w/V.

3. The kit according to one of the preceding claims, **characterized in that** said acidic compound is an inorganic acid.

4. The kit according to one of the preceding claims, **characterized in that** the acidic compound is comprised in the group consisting of sulfuric acid, fluoroboric acid, hydrochloric acid, citric acid, ascorbic acid and nitric acid.

5. The kit according to one of the preceding claims, **characterized in that** said liquid organic compound is ethyl acetate.

6. The kit according to one of the preceding claims, **characterized in that** said first container comprises an aqueous solution comprising from 15 to 60 % by weight of said acidic compound and from 1 to 50 % by weight of said organic compound.

7. The kit according to one of the preceding claims, **characterized in that** said first container comprises viscosifiers to allow its application by spreading.

8. A method for cleaning the yellow/brown line formed at the waterline on plastic material walls of a swimming pool; said method being **characterized in that** it comprises in sequence:
- a first application operation, in which (i) a solution of an acidic compound having a pKa at 25°C less than or equal to 5 at a concentration greater than or equal to 15% by weight and/or (ii) a liquid organic compound belonging to the class of ketones or glycols or acetates is applied on an application area including a yellow/brown line formed on plastic material walls of a swimming pool, and
- a second application operation, in which a solution comprising sodium hypochlorite at a concentration greater than or equal to 3% w/V is applied over said application area.
